# EUROPEAN PATENT SPECIFICATION

(11) **EP 3 302 691 B1**
(45) Date of publication and mention of the grant of the patent: **24.07.2019**
(21) Application number: 16730144.9
(22) Date of filing: 02.06.2016
(51) Int. Cl.: A61N 1/36, A61F 2/00, A61B 5/04, G06F 3/01, A61N 1/04, A61B 5/00

(54) **NON-INVASIVE MOTOR IMPAIRMENT REHABILITATION SYSTEM**
NICHTINVASIVES SYSTEM ZUR REHABILITATION EINER MOTORSTÖRUNG
SYSTÈME NON EFFRACTIF DE RÉÉDUCATION DE DÉFICIENCE MOTRICE

(30) Priority: 02.06.2015 US 201562169810 P
(43) Date of publication of application: 11.04.2018
(60) Divisional application: 19174422.6
(73) Proprietor: Battelle Memorial Institute, Columbus, OH 43201 (US)
(72) Inventor: BOUTON, Chad E., Columbus, Ohio 43201 (US)
(74) Representative: Kador & Partner PartG mbB
(86) International application number: PCT/US2016/035503
(87) International publication number: WO 2016/196784

(56) References cited:
- WO-A2-2014/089266
- US-A1- 2010 286 748
- US-A1- 2011 307 079
- US-A1- 2014 200 432

## Description

### BACKGROUND

The present disclosure relates generally to systems, methods and devices for motor impairment rehabilitation.

Many millions of people suffer from some motor impairment. For example, it is estimated that worldwide, 10 million people are left disabled following a stroke each year. People also suffer from brain injury, failed back surgery or spinal cord injury. These injuries can result in motor impairment by damaging the link between the brain and the muscles of the body which are used for movement. For example, neurons in the brain may die, or the nerves between the brain and the muscle are severed. These disrupt the paths by which electrical signals travel from the brain to neuromuscular groups to effectuate coordinated muscle contraction patterns. Impairment of motor function affects a person's lifestyle and can create hardship for the person's family.

It would be desirable to provide systems and methods for rehabilitating a patient following a motor impairment injury. These systems and methods could, over time, retrain neural pathways of a person, or help a patient regain strength or dexterity in an affected limb following an injury. US2010286748 discloses a method and system for connecting an impaired nervous system of a patient to a destination target of a muscle, group of muscles or robotic device.

### BRIEF SUMMARY

The present disclosure relates to systems and methods for motor impairment rehabilitation. EEG and neuromuscular stimulation are used to promote neuroplasticity, improving retraining and recovery of affected areas of the brain, and ultimately improve the patient's strength and/or dexterity.

In one aspect, a device for rehabilitating a patient with motor impairment includes: receiving electrical signals from electrodes positioned on or in the patient's head; determining an intent to move a specific body part of the patient from the electrical signals; determining a focus level of the patient from the electrical signals; and based on the intent to move and focus level, delivering electrical stimulation through sleeve electrodes of a sleeve positioned on the specific body part of the patient to assist in the desired movement of the specific body part.

The intent to move may be calculated based on a change in amplitude of the beta frequency band in the received electrical signals, for example a decrease in the amplitude of at least 5% for a time of at least one-tenth second. The beta frequency band may be in the range of 16-31 Hz. The focus level may be determined by a change in amplitude of the gamma frequency band in the received electrical signals, such as an increase in the amplitude of at least 5% for a time of at least one-tenth second. The gamma frequency band may be 32 Hz or more. The intent to move may include any one of: an imagined movement; an attempted movement; or an executed movement.

The intent to move and/or the focus level are generally calculated after the patient goes through a calibration period of about five seconds to about 20 seconds to determine a baseline by which changes are measured.

The device may further comprise determining that the patient is focusing on a desired movement of the specific body part. The amplitude of the delivered electrical stimulation to the specific body part can be a function of a feature of the received electrical signals. Such features may include of the signal amplitude, the amplitude of the signal in a given frequency range, the amplitude of the signal in a wavelet scale, the firing rate, and combinations thereof.

Other devices may further include: tracking an eye movement of the patient with an eye tracking device; and modifying the focus level based on the electrical signals from the electrodes and the tracked eye movement. The eye tracking device may include a camera connected to the headset. The determined focus level of the patient is modified based on whether an eye of the patient is focused on the specific body part of the patient.

Additional devices may further include engaging the patient with a rehabilitative videogame that instructs the patient to move an impaired body part. The difficulty level of the videogame can be increased if the focus level is below a predetermined threshold.

Some devices may further include: during a first rehabilitative session, calculating a stimulation factor based on the electrical signals; prior to a second rehabilitative session, reducing the calculated stimulation factor; and delivering the electrical stimulation based on the calculated stimulation factor.

Some devices may further include providing a visual or aural indication that the patient is not focused if the focus level is below a predetermined threshold,

The received electrical signals from the patient's brain can be received from implanted electrodes implanted within the patient. In other embodiments, transcranial direct current stimulation (tDCS) is used with the patient.

In another aspect, devices for rehabilitating a patient with motor impairment include: using both electroencephalogram (EEG) to receive electrical signals of a patient; determining an intent to move a specific body part of the patient from the electrical signals; determining a focus level of the patient from the electrical signals; and based on the intent to move and focus level, delivering electrical stimulation through sleeve electrodes of a sleeve positioned on the specific body part of the patient to assist in the desired movement, and delivering transcranial direct current stimulation (tDCS).

These and other non-limiting aspects of the disclosure are more particularly discussed below.

### BRIEF DESCRIPTION OF THE DRAWINGS

The following is a brief description of the drawings, which are presented for the purposes of illustrating the exemplary embodiments disclosed herein and not for the purposes of limiting the same.
**FIG. 1** diagrammatically illustrates one embodiment of the methods of the present disclosure, and a system for practicing the methods.
**FIG. 2** is a diagram illustrating the methods used in the present disclosure for decoding a neural signal to determine desired movements by a user.

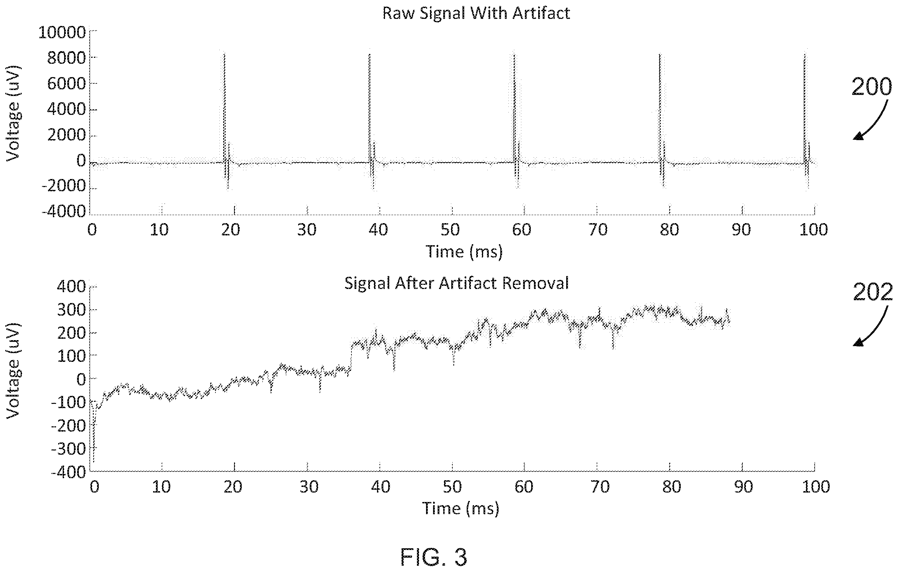

**FIG. 3** illustrates a neural signal before and after artifact removal. In the "before" graph at top, the y-axis is voltage in microvolts, with the scale ranging from - 4000 to +10,000 µV at intervals of 2000 µV. The x-axis is time, with the scale ranging from 0 milliseconds (msec) to 100 milliseconds at intervals of 10 msec. In the "after" graph at bottom, the y-axis is voltage in microvolts, with the scale ranging from -400 to +400 µV at intervals of 100 µV. The x-axis is time, with the scale ranging from 0 milliseconds (msec) to 100 milliseconds at intervals of 10 msec. It can be seen that the "before" time has been processed such that the "end" time is of a shorter duration (about 10% shorter), which is due to removal of signal artifacts.
**FIG. 4A** is a diagram indicating single-motion decoders, which provide simple binary output (yes/no) to identify a desired movement.
**FIG. 4B** shows a discrete multiclass decoder and a movement effort decoder. The multiclass decoder can be considered an amalgamation of the single-motion decoders illustrated in **FIG. 4A****.** The movement effort decoder outputs a measure of the focus level being applied to attain the desired movement.
**FIG. 5** is a diagram illustrating additional features of the methods described herein, also including boxes showing how the decoders are trained to identify desired movements from a neural signal.
**FIG. 6** shows an exemplary graphical display used in the methods herein.
**FIG. 7** shows a spatial pattern obtained from a 96-microelectrode array.
**FIG. 8** is a plan view of one embodiment of a neural sleeve that can be used for practicing the methods of the present disclosure.
**FIG. 9** is an exemplary photograph showing two neural sleeve devices according to the embodiment of **FIG. 8** which are wrapped around a patient's arm region in preparation for neuromuscular stimulation.
**FIG. 10** is diagram of another exemplary embodiment of a neural sleeve. In this embodiment, conductive pathways extend from two different connectors. The fingers extend in the same direction, and taper towards a center axis.

### DETAILED DESCRIPTION

A more complete understanding of the methods and apparatuses disclosed herein can be obtained by reference to the accompanying drawings. These figures are merely schematic representations based on convenience and the ease of demonstrating the existing art and/or the present development, and are, therefore, not intended to indicate relative size and dimensions of the assemblies or components thereof.

Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function.

The systems and methods described herein contemplate the use of electrical stimulation in rehabilitating a patient following a motor impairment injury. Electrical stimulation may help rehabilitate a patient in various ways. For example, electrical stimulation applied to specific muscles or nerve may help the patient effectuate an intended movement. As another example, electrical stimulation applied to a patient's brain appears to increase neuroplasticity in a patient's brain. Thus the patient is able to more easily learn / relearn specific movements or to compensate for the injury.

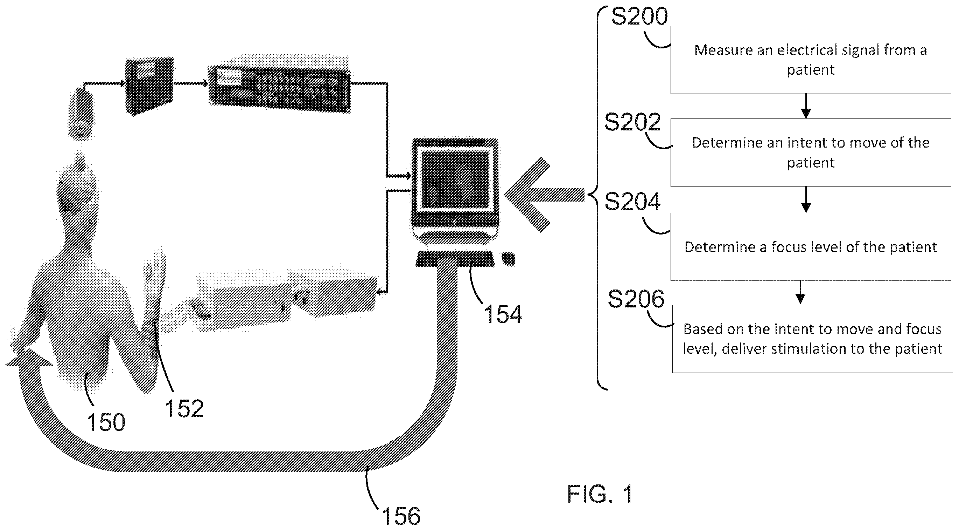

With reference to **FIG. 1****,** as a basic overview, a system for neural rehabilitation of the present disclosure includes electrodes for sensing brain signals from the brain of a user **150.** Computer or processing unit **154** is connected to receive neural signals from the electrodes, and to output control signals to the transcutaneous neurostimulation sleeve **152,** and further programmed to determine a volitional intent of the subject based on the received neural signals and to generate the output control signals to implement that volitional intent. The computer **154** receives the user's neural activity as an input, and determines the desired motion. Based on the analysis done by computer **154,** an electrical stimulation pattern is delivered to the user through neural sleeve **154.** The computer **154** may also calculate an electrical stimulation pattern to be delivered to the user's brain, as illustrated by arrow **156.** As noted above, the electrical stimulation delivered to both the neural sleeve **154** and the user's brain may have rehabilitative effects following a motor impairment injury.

In this regard, the present systems monitor the neural activity of the patient. The patient is asked to move an affected body part (e.g. a limb such as an arm or a leg). The computer monitors the patient's neural activity to identify the "signal" of the desired movement. It should be understood that this reference to the desired movement includes a movement being imagined, attempted or executed, depending on the physical capability of the user. It does not require actual movement to occur. The system then provides feedback on how well the patient is doing in generating the neural signals that would produce the desired movement, through electrical stimulation to the affected body part and to the brain.

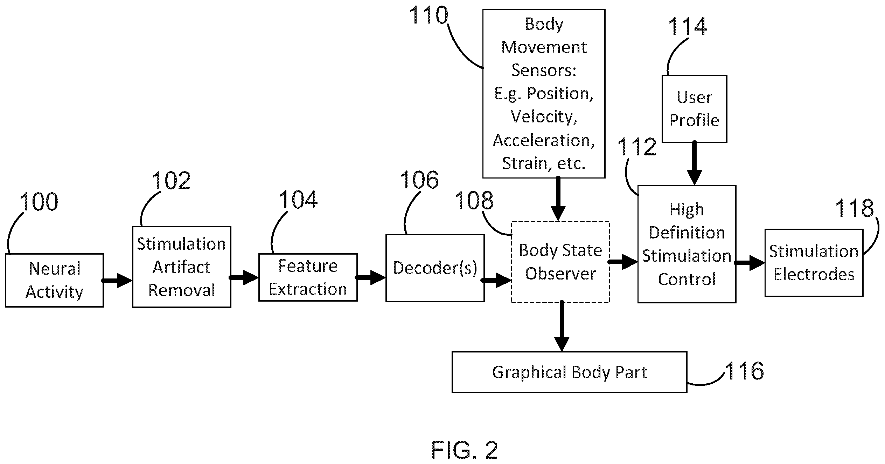

**FIG. 2** illustrates a flowchart / algorithm for determining the imagined motion from a user's neural activity and translating that into an electrical stimulation pattern that can be transmitted. First, the neural activity of the user **150** is measured in operation **100** to obtain a neural signal. Noise artifacts are then removed from the neural signal in operation **102.** To determine the imagined movement, one or more features are extracted from the measured neural signal in operation **104.** Subsequently, the extracted feature(s) are sent to decoders in operation **106.** The decoders determine the imagined movement. That output is then sent to a body state observer **108,** which is a model of the various parts of the user's body. Inputs from body movement sensors **110** may also be taken into account in the body state observer **108,** which is used to predict future movements that need to be made to obtain the imagined movement. In operation **112,** high definition stimulation control is used to calculate the stimulation pattern that is needed to obtain the imagined movement. A user activation profile **114** containing information on the user's reaction to various stimulation patterns can be used here to customize the resulting stimulation pattern / signal that is determined. That stimulation pattern is then sent to stimulation electrodes **118,** which stimulate the appropriate part of the body part (e.g. muscle groups, etc.). Alternatively, during training, the system calculates the effect such stimulation would have, and displays this in the form of a graphical body part **116,** to provide feedback to the user. This algorithm repeats at a high rate, permitting continuous real-time updating of the stimulation signal to the target. Thus, it should be understood that the methods described herein may be performed either continuously or intermittently.

During rehabilitation, the goal is to train the patient to increase the speed and/or strength of the desired neural signal, so that over time the desired movement is more easily accomplished by the patient. The computer / software looks for indications in the neural signals that the patient is trying to evoke the particular movement command in the motor areas of the brain. In particular, the computer / software gauges the (1) focus level and (2) the intent to move, both of which are key to a successful rehabilitation session, as the patient tries to move the affected body part. The computer / software will also provide stimulation to the affected body part to assist the patient with the targeted movement. Advantageously, by linking the focus level and the intent to move with the level of the stimulation provided to the affected body part, neuroplasticity is promoted in the brain. This increases the effectiveness of the rehabilitation and decreases the total time required for rehabilitation therapy.

Returning to **FIG. 1****,** the methods implemented by the software are described on the right-hand side. First, as shown in step **S200,** the patient's neural activity is measured in response to instructions to imagine a desired movement. The neural activity is essentially a set of electrical signals that is generated by the brain and measured for input into the computer. This measurement may be done by any suitable technique. For example, electroencephalography (EEG), a noninvasive technique, may be used. In EEG, electrodes are placed on a scalp of patient **150** to measure the electrical activity. Invasive techniques (electrodes placed beneath the scalp) are also contemplated for use. For example, a "Utah array" of electrodes, such as that made by Blackrock Microsystems, may be used. The Utah array can have up to 96 electrodes. Also contemplated is the use of a "Michigan array" of electrodes, such as that made by NeuroNexus. Electrocorticography (ECoG) may also be used. In ECoG, electrodes are placed directly on the exposed surface of the brain to record electrical activity. Hemiparesis or hemiplegia, weakness or complete paralysis of one side of the body, can occur in stroke patients. In particular embodiments, it is contemplated that the electrodes are placed on the hemisphere of the brain that is opposite the paralyzed side of the body.

These electrode arrays record "brain waves," more particularly neural signals which are representative of a varied set of mental activities. Neural signals include electrical signals produced by neural activity in the nervous system including action potentials, multi-unit activity, local field potential, ECoG, and EEG. The neural signals from each electrode can be sampled at rates of at least 10 kHz, providing a robust stream of data (though not all of this data needs to be used). These neural signals are sent wirelessly or, alternatively, through a wired connection, to a neural signal processing device for processing of the neural signals.

After measurement, the electrical signals indicating the patient's neural acitivity are analyzed. The electrical signals are processed to determine, for example, the signal amplitude, the amplitude of the signal in a wavelet scale, the firing rate, or power levels in different frequency bands, or changes therein. Based on these features, the patient's intent to move is determined in operation **S202.** The intent to move may be determined, for example, by finding a change in the amplitude of the beta frequency band (16-31 Hz) before a movement is imagined. More specifically, the amplitude will decrease by at least 5% and maintain that decrease for a period of at least one-tenth (0.1) of a second. In more particular embodiments, the amplitude will decrease by at least 10%, or by at least 20%, with higher decreases in amplitude reflecting greater certainty of an intent to move. The decrease in amplitude may be maintained for a period of at least 0.1 seconds, and up to 1 second.

The patient's focus level is also determined in operation **S204.** The focus level can be determined, for example, by measuring a change in amplitude in the gamma band (32 Hz or higher, up to 100 Hz), which is indicative of an increased focus level. More specifically, the amplitude will increase by at least 5% and maintain that decrease for a period of at least one-tenth (0.1) of a second. In more particular embodiments, the amplitude will increase by at least 10%, or by at least 20%, with higher increases in amplitude reflecting a greater focus level. The increase in amplitude may be maintained for a period of at least 0.1 seconds, and up to 1 second. As will be explained further herein, the decoders can also be used to determine that the patient is focusing on the correct movement.

Based on the intent to move and focus level, electrical stimulation is delivered to the patient in operation **S206.** This electrical stimulation can be delivered to at least two different locations. First, the body part that the patient is trying to move can be stimulated. This provides some feedback to the patient. Second, the patient's brain can also be stimulated. In particular, transcranial direct current stimulation (tDCS) raises the excitability of neurons in the brain, facilitating healthy neuroplasticity and rehabilitation, along with the stimulation to the targeted body part.

If desired, the system can vary the amplitude of the delivered stimulation (electrical current level) as a function (proportionally, linearly, or non-linearly) of the neural signal/features being monitored, either to the affected body part or to the brain. The neural signal/features being monitored may include, for example, the gamma or beta frequency bands of the patient. Therefore, when the patient tries harder to achieve the desired movement, the stimulation level can be varied as desired. Over time, the strength of the variance can be decreased by the system automatically as the patient regains their own natural strength and motor abilities. This neuromuscular stimulation will also help reduce or prevent muscle atrophy during the rehabilitation period while the patient regains strength.

In particular embodiments, prior to beginning rehabilitation, the patient undergoes a calibration period. During this time, the patient is asked to clear his mind. The neural signal is then measured to obtain normal power levels in the various frequency bands. This creates a baseline for future analysis. The calibration period is usually for a time of about five second to about 20 seconds.

Returning now to **FIG. 2****,** it may be helpful to discuss each action that occurs in interpreting the neural signals received and generating electrical stimulation for both the body part that is being rehabilitated and the brain of the patient.

As previously mentioned, the neural activity of the user **150** is measured in operation **100** to obtain a neural signal. Next, the neural signal is processed **102** to obtain a "clean" signal. In this regard, for most purposes, it is desirable for each electrode to record the signal from a given neuron, rather than a set of given neurons. The brain is very busy electrically, and the presence of other neurons in the vicinity of these delicate and sensitive electrodes can create noise that obscures the desired signal. The signals actually detected by the electrode array are first amplified and then filtered to remove noise outside of a frequency band of interest (e.g. 0.3 Hz to 7.5 kHz). The signal may be processed in analog or digital form. Examples of useful analog filters include an 0.3Hz 1st order high pass filter and a 7.5kHz 3rd order low pass filter. An example of a digital filter is a digital 2.5kHz 4th order Butterworth low pass filter.

Part of the processing **102** involves artifact removal. Artifact removal is used to "clean up" the neural activity data and results in improved processing. Artifacts in the data may have been caused by, for example, electrical stimulation in the body limb (e.g. forearm) whose movement is desired. **FIG. 3** shows a signal before artifact removal **200,** and shows the "same" signal after artifact removal **202.** Identification of an artifact may be accomplished by, for example, by detecting a threshold crossing in the signal data. Here, for example, the artifacts are the extremely high peaks up to 8000 µV at periodic rates of ∼20 msec. The threshold can be fixed or dynamically calculated, and for example can be modified based on factors already known to the processor such as when electrical stimulation is delivered through the neurostimulation sleeve. A set time window of data may then be removed around the detected artifact. The data is then realigned (e.g. voltage-wise) and then stitched back together (e.g. time-wise). For example, as shown here, the time window is 2.5 milliseconds, based on the system's recovery period for an amplifier. The five peaks are thus removed, and the remaining signal is shown on the bottom. As seen here, once the artifacts are removed, the signal is of much smaller magnitude but contains useful information.

Of course, when data is being measured on multiple channels (e.g. from a 96-channel microelectrode array), the artifact should be removed on each channel. One common method of artifact removal is to determine the average from all or most of the channels, and then subtract the average from each channel. Alternatively, the stimulation signal can be shaped in such a way that artifacts on certain frequencies may be prevented or reduced. In other embodiments, the artifacts can be planned somewhat. For example, the electrical stimulation delivered through the neurostimulation sleeve **152** could have a known shape, such as a square pulse. These will aid in removing artifacts from the neural signal.

Next, in operation **104** of **FIG. 2****,** features are extracted from the neural signal. "Feature" is the term given to various pieces of data, either from each electrode individually or some/all electrodes considered as a group, that can contain useful information for determining the desired movement. Examples of such features include: signal amplitude, amplitude of the signal in a given frequency range, amplitude of the signal in a wavelet scale, or a firing rate. Here, a firing rate may refer to the number of action potentials per unit of time for a single neuron. Again, the extracted features provide useful information about the neural network being monitored.

One particular set of features is obtained by applying a wavelet decomposition to the neural signals, using the 'db4' wavelet and 11 wavelet scales, as described in Mallat, S.G., A Wavelet Tour of Signal Processing, Academic Press (1998), (ISBN 9780080922027). Scales 3 to 6 are used to approximate the power in the multi-unit frequency band. The mean of wavelet coefficients for each scale of each channel (of the 96-electrode array) is taken across 100 milliseconds of data. A 15-second sliding window is used to calculate the running mean of each wavelet scale for each channel. The 15-second mean is then subtracted off of the mean wavelet coefficients of the current 100 ms window. The mean subtraction is used in order to account for drift in the neural signal over time. The coefficients of scales 3 to 6 are standardized per channel, per scale, by subtracting out the mean and dividing by the standard deviation of those scales and channels during training. The mean of each channel is then taken across scales. The resulting values are then used as features to be inputted to the decoder(s) running in real-time.

In some applications, a Fast Fourier Transform (FFT) may be used to extract features. Advantageously, an FFT may be used for obtaining power information. Additionally, a nonlinear or linear transform may be use to map the features to N-dimensional space (e.g. by use of a radial basis function). This may be useful when a desired movement can manifest itself in the form of multiple different electrical signals from the electrodes, so that the system can recognize any of those different signals.

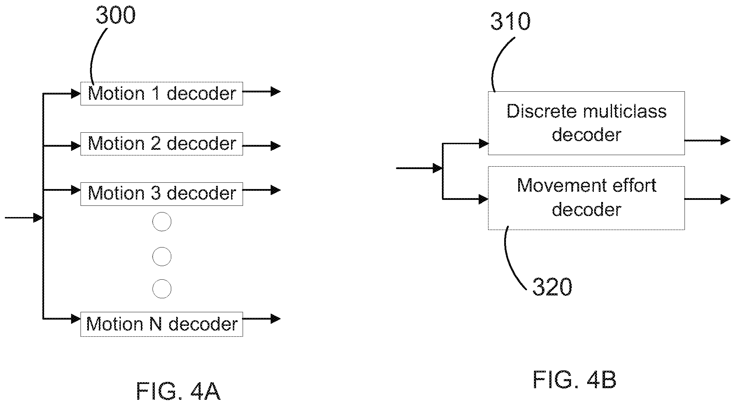

Next, the extracted features are sent to one or more decoders **106** that associate the features with a particular action, movement, thought, or so forth. It is contemplated that decoders can be implemented in at least two different ways. First, as illustrated in **FIG. 4A****,** the extracted features may be sent as input to individual decoders **300.** Each decoder has previously been "trained" to associate certain features with a particular movement. Examples of such decoded motions include: individual finger flex or extension; wrist flex or extension; radial deviation; forearm supination or pronation; and hand open or close. Each decoder then outputs a binary yes/no output as to whether its particular movement has been identified. Advantageously, use of the decoders allows for a determination of related, simultaneous movements. For example, the decoders may determine that the user is imagining a hand is closing with either the arm moving or not moving. In addition, each decoder may determine a level of effort associated with its motion. Also, the training dataset used to build the decoder may only maintain a certain amount of history, so that the decoder may adapt to changes in the neural signals over time.

Alternatively, as illustrated in **FIG. 4B****,** the decoders can be organized in the form of a discrete multiclass decoder **310** that is used in parallel with a movement effort decoder **320.** The discrete multiclass decoder determines the motion(s) that is being imagined. For purposes of this disclosure, the multiclass decoder can be considered as a software module that receives the features as input, and can output any one of multiple desired movements. The movement effort decoder determines the level of movement effort of the identified movement(s). Any decoded signal may be linearly or nonlinearly mapped to determine a signal delivered to the target. In general, the system can determine both the user's movement intention before the physical movement begins, and also during movement as well. Again, it should be noted that the neural signals from the user are for movements that may be imagined or attempted, as well as actually performed.

Next, the output of the decoders is sent to a "body state observer" **108.** The body state observer is a physics-based dynamic model of the body (e.g. including body parts such as arm, hand, leg, foot, etc.) implemented as software. The body state observer takes the desired forces, torques or so forth as inputs and continuously updates and outputs the velocity and position estimates of the actual body part(s). The body state observer can also accept data from body movement sensors **110** as input. Such sensors may provide information on the position, velocity, acceleration, contraction, etc. of a body part. For example, sensors could provide information on the position of the elbow relative to the shoulder and the wrist, and how these body parts are moving relative to each other. In addition, the body state observer has a "memory" or a history of the feedback previously provided to the user.

The use of a body state observer is considered to have at least two advantages. First, the outputs of the body state observer can be used to continuously or dynamically change the stimulation patterns being outputted to the neurostimulation sleeve, to account for changed circumstances. For example, if the stimulation electrodes are transcutaneous, they can move with respect to their target muscles as the joints move (e.g. pronation/supination). The stimulation pattern given through the stimulation electrodes can thus be modified according to the relative shift between electrodes and muscles. In other words, the stimulation pattern may be dynamically changed (or held constant) based on a determined body state. This dynamic change in the stimulation pattern may be referred to as electrode movement compensation (EMC). In one example of EMC, a stimulation pattern may be changed based on whether a user's palm is up or down. Second, for transcutaneous electrodes and even implanted electrodes, the force or torque at a given stimulation current is often a function of joint position, velocity, or so forth. As the observer predicts joint position and velocity, the stimulation current can be adjusted accordingly to maintain a force or torque desired by a user.

Examples of body states considered by the body state observer include palm up or palm down; arm moving or not moving; a flexing, extension or contraction of a wrist or other body part; and positions or movements of joints.

As another example, the body state observer can use the decoder outputs to estimate angular force at the joints corresponding to the desired motion using a model of the hand and forearm with 18 degrees of freedom. The model estimates the force caused by the contraction of the muscle, the force opposing the muscle contraction due to the anatomy of the hand and forearm, a damping force, and the force of gravity. The output of the model is an estimate of the position of the hand and forearm in real-time, taking into account the history of the stimulation provided to the forearm in order to estimate a current position of the hand and forearm.

Based on the output of the body state observer, the electrical stimulation pattern that is to be sent to the neurostimulation sleeve is determined by an encoding algorithm that generates the appropriate spatiotemporal patterns to evoke appropriate muscle contractions. The present disclosure permits high definition stimulation. In high definition stimulation, the simulation pattern to the electrodes is "continuously" updated. For example, the stimulation pattern to the electrodes is updated once every 0.1 seconds (e.g. 10 Hz). However, shorter and longer update times are also contemplated; in fact, speeds up to 50 Hz are contemplated. As discussed above, the simulation pattern is provided based on the decoded motion(s) and is adjusted based on body states determined by the body state observer. To create a smoother motion, a nonlinear or linear mapping may be applied to the output of the decoder(s). The user's particular user activation profile **114** can also be used to modify or determine the electrical signal that is sent to the target. In this regard, different patients need a different stimulation pattern to obtain the same movement of the target. Advantageously, this allows for delivery of a more effective stimulation pattern based on the individual characteristics of a user. The stimulation pattern is then sent to the electrodes **118** to be transmitted to the muscles.

Additionally, in high definition stimulation, multiple signal patterns may be interleaved (e.g. by multiplexing) if more than one motion is desired (e.g. a compound motion). For example, the stimulation pattern needed to lift the arm may be directed to different muscles than those for rotating the wrist. Interleaving permits multiple stimulation patterns to be combined into a single stimulation signal sent to the neuromuscular sleeve, so that multiple movements can occur at the same time. Again, this permits the body limb to move more naturally. In addition, advantageously, interleaving prevents electric field patterns created by one stimulation pattern from interfering with electric fields created by another stimulation pattern. This increases the number of complex motions that the system is capable of. However, there is a practical limit to the number of stimulation patterns that may be interleaved due to the fact that when the pulse rate for a single simulation pattern becomes too low (e.g. less than 10 pulses per second), muscle twitches will start to become noticeable and movement smoothness becomes undesirable. Still, interleaving is very effective, and allows for multiple movements to be performed simultaneously (e.g. in a compound movement). This could not be achieved with only a single simulation pattern.

In addition, motions may be sequenced. One example of this in a natural system is a central pattern generator, which produces rhythmic patterned outputs without sensory feedback. The software / systems of the present disclosure can mimic central pattern generators by producing a repeatable sequence of events, for example to return a targeted body part to an initial state. Another example of sequenced motions is a functional series of motions. Examples of functional series of motions include: teeth brushing, scratching, stirring a drink, flexing a thumb, cylindrical grasping, pinching, etc. These motions allow for manipulation of real-world objects of various sizes.

As a result of the stimulation **118,** the body part moves as imagined by the user, which can serve as feedback to the user. The electrical stimulation can be provided in the form of current-controlled, monophasic pulses of adjustable pulse rate, pulse width, and pulse amplitude which can be independently adjusted for each channel. This cycle repeats continuously. The stimulation signal / pattern sent to the electrodes can be changed continuously through each cycle if needed, or can be maintained, in order to complete the imagined movement. The software can monitor either or both the neural activity and the motion of the target as detected by the sensors. It is noted that the neural signal may not simply remain constant from the beginning of the imagined movement until the end of a desired movement. Rather, for example, as an arm is moving, the neural signal will change. This is because the body is providing dynamic information to the brain on features such as the position and velocity of the moving arm. The software can distinguish between these changes in neural activity based on time. Alternatively, the stimulation signal sent to the target may be actively changed due to changes in the body state, for example due to the shift in the electrode position relative to their targeted muscle groups as a body limb moves. Desirably, the decoder is also robust to context changes (such as arm position and speed).

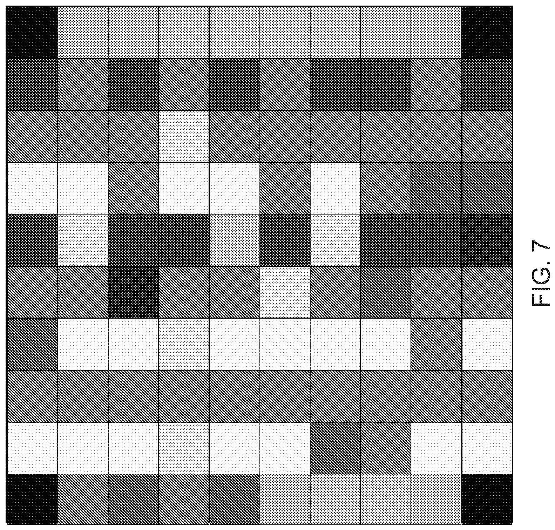

In this regard, it is noted that the intent to move and the focus level that are used in the rehabilitation can be implemented in the form of decoders. As discussed above, the "intent to move" is a measure of what movement the patient / user is trying to make with a given body part. The "focus level" is a measure of how hard the patient / user is trying to make the movement. It is also contemplated that a decoder can be used to determine that the patient / user is focusing on the correct movement, as opposed to focusing intently on something else. In this regard, it is possible to examine different spatial patterns of the electrode arrays. **FIG. 7** is an example of a spatial pattern obtained from a 96-electrode array. This is a 10x10 array, with the four corners empty and not representing an electrode, and each other square representing the output of a given electrode. The different textures in each square represent a given amplitude of that electrode. Different types of movements, for example a wrist movement versus a finger movement, have different spatial patterns. During rehabilitation, whether the user is focusing on the desired movement can be identified using a decoder as well. Keep in mind, the movement imagined by the user may not be the desired movement that the system is looking for. For example, the system may ask the user to focus on moving the thumb (i.e. the desired movement), but the user actually focuses on moving the index finger (i.e. the imagined movement). In this case, the index finger could be stimulated to move, providing visual feedback that the user is not correctly focusing on the desired movement. However, alternatively, the fact that the user is not focusing on the desired movement can be detected, and the stimulation signal can be set to zero, and the lack of movement is another visual indication to the user.

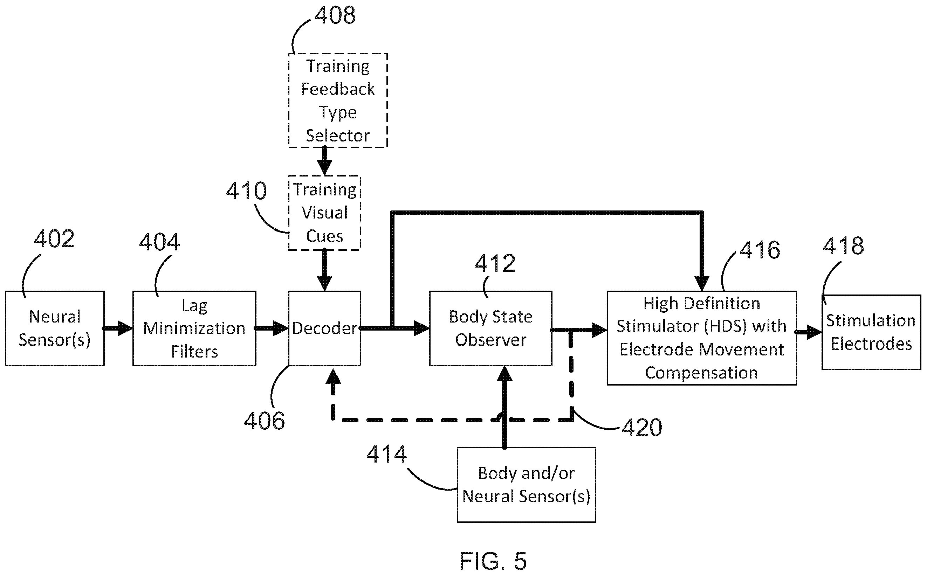

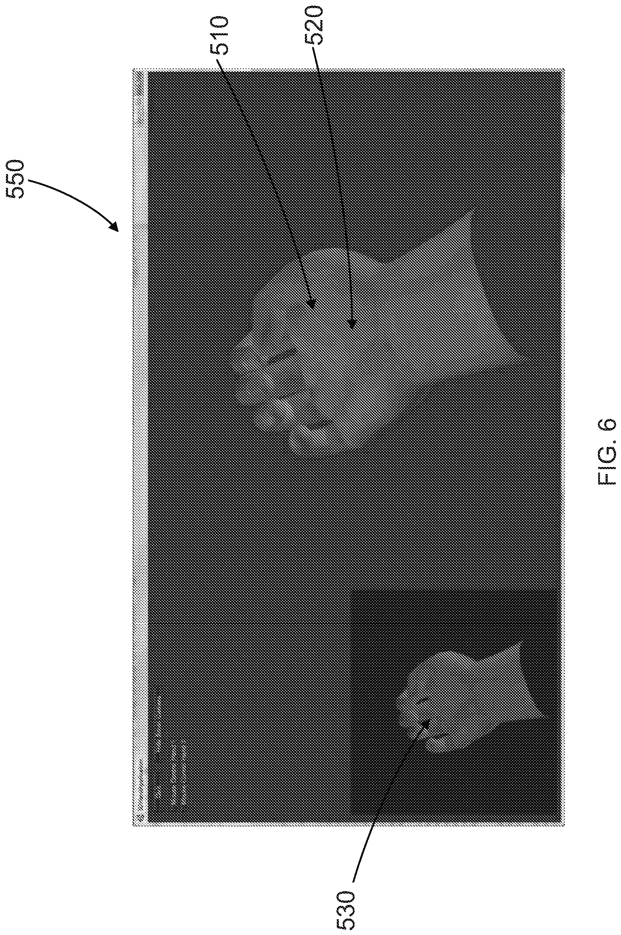

In order to successfully operate, the software must be trained in decoding the neural signal to determine the desired movement. **FIG. 5** shows a flowchart related to such training. This flowchart is very similar to that of **FIG. 2****.** Neural sensors **402** record a neural signal which is sent to lag minimization filters **404.** The lag minimization filters **404** use *a priori* statistical information about the neural sensor signals and use the least amount of history to filter the signals (minimizing lag and attenuation that fixed time window filters impose). In addition, during decoder training, a training feedback type selector **408** operates in conjunction with training visual cues **410.** The visual cue of operation/component **410** may be provided in the form of a large graphical body part **510** (shown in **FIG. 6**). In this way, the user may attempt or imagine a movement that the user is being shown, for example, by large graphical body part **510,** and then the user will produce neural signals that are useful in training a decoder **406** to identify the movement being shown. The dots for training feedback type selector **408** and training visual cues **410** indicate that these boxes are used only for training, not during actual operation of the system.

In one related example of decoder training, a user may close his hand with his arm either moving or not moving while the decoders continuously search for patterns. The decoder **406** receives inputs from both the lag minimization filters **404** and the training visual cues **410.** In addition, during decoder training, the decoder **406** also receives an input from a body state observer **412,** which is shown by dashed line **420.** The body state observer itself receives input from body and/or neural sensors **414.** The decoder thus receives feedback that helps it to identify the signals indicative of a particular movement. Stimulation is also provided via stimulator **416** to electrodes **418,** so that any change in neural activity due to the desired movement is reflected in the input from the neural sensors **402.**

Additionally, in decoder training, suitable machine learning techniques may be employed. For example, support vector machine techniques may be used. During training, cues (e.g. visual, aural, etc.) may be presented to the user while neural data is collected. This data is collected for various positions over many different contexts in which the user might be thinking about a certain motion, to provide better training data. For each point in time that a feature is calculated, the corresponding cue may be recorded in the cue vector. After training, a decoder may be built using the feature matrix and cue vector. A decoder is typically built by solving for weights matrix 'w' and bias '┌' using suitable machine learning techniques to perform classification (e.g. discrete output) or regression (e.g. continuous output). Examples of methods that can be used to build the decoder include: Linear Discriminant Analysis, Least Squares Method, and Decision Trees such as Random Forest Type. The inputs and/or outputs to the decoder can be filtered (e.g. smoothed over the time domain) as well by using lowpass, high-pass, band-pass, Kalman, Weiner, etc. type filters. Multiple decoders may be built (e.g. individual motion decoder, discrete multiclass decoder, movement effort decoder, etc.). Individual movement decoders or a movement effort decoder can be used in real-time to decode the intended force 'F' by using *F* = *A* ∗ *w* - Γ where 'A' is the vector of features for the current point in time.

Additionally and advantageously, decoders are trained to recognize both discrete type motions and rhythmic type motions. A discrete type motion is generally a single movement. In contrast, rhythmic type motions involve multiple, repeatable "sub-motions." Rhythmic type movements are used in daily activities such as teeth-brushing, cleaning/scrubbing, stirring liquids, and itching or scratching. Notably, when a user imagines a rhythmic movement, the user's neural activity pattern is different than when the user is imagining a non-rhythmic movement, and identifying these patterns sooner can help the resulting movements be more natural.

In one example, three stimulation levels corresponding to a low, medium, and high evoked force response are provided to a user. A piecewise-linear interpolation is used to construct a mapping for decoder output to stimulation amplitudes for each motion. During real-time decoding, this mapping allows for smooth physical transitions from one movement to the next, controlled by the modulation of intracortical signals. A single point calibration combined with linear interpolation is insufficient due to the nonlinear response of the arm to electrical stimulation of varied amplitudes. At lower intracortical modulation levels, the stimulation produced would not be strong enough to evoke the desired motion. The three point calibration facilitates the initiation of the motion in the cases of lower modulation levels.

Referring now to **FIG. 6****,** the display of a graphical body part can be advantageously used for decoder training and real-time feedback to the user. Here, a graphical body part **510** is displayed on a graphical user interface (GUI) **550.** The graphical body part **510** may be in the form of a large hand **520** and/or a small hand **530.** In one example, the small hand **530** is used to show the user the desired movement. The user may then attempt to perform the movement by thinking about it. The large hand **520** provides feedback to the user on how well s/he is doing. For example, the small hand **530** can be controlled by an executable macro that provides cues to the user, while the large hand **520** is controlled by the body state observer **108.** In this way, the user is provided with both visual feedback (from the large hand **520**) as well as electrical simulation feedback (from the electrodes). The data collected is used to "train" the decoder for the particular movement. This is repeated for multiple different potential movements. Besides a virtual hand, any other virtual body part (e.g. a virtual leg) may be created.

In some embodiments, while the user is attempting or imagining a movement cued by the GUI **550,** electrical simulation may be applied to the user in order to help evoke the cued movement. Therefore, the electrical stimulation operates both to provide feedback to the user and evoke the cued movement.

Additionally, a movement depicted on GUI **550** may be a rhythmic type movement (in contrast to a discrete type movement). Rhythmic type movements are used in daily activities such as teeth-brushing, cleaning/scrubbing, stirring liquids, and itching or scratching.

To test the decoders, the user is asked to imagine the movements again, and the system is used to decode their thoughts in real time. While continuously decoding, the system also stimulates the electrodes on the limb to evoke the decoded movement, providing feedback to the user.

The rehabilitation software can take the form of games/tasks running on a computer or mobile device (ipad, iphone, mobile phone, etc.) that interfaces with the system. For example, the software can include visual 'coaching' through the use of a virtual hand, which is a 3D animated hand that includes a physics-based model allowing it to more closely emulate an actual human hand. This makes the rehabilitation session more realistic and intuitive. The virtual hand movement can excite mirror neurons in the brain (important in learning or relearning movements) while promoting neuroplasticity and healing/recovery. The user could also use the virtual hand movement or other moving elements in the game/task to achieve a goal, such as pushing, pulling, or otherwise manipulating a virtual object on the screen of the computer / device.

It is contemplated that the rehabilitation will include very specific joint movements (hand, leg, face, etc.) affected by stroke or other injury, and each movement of interest can be stimulated using the high definition stimulation sleeve technology discussed further herein. The system can sequence through several desired movements one at a time and allow the user to focus on each movement. For each movement, the system may automatically stimulate the precise joint movement being shown by the virtual body part of **FIG. 6****.** Furthermore, the user will focus on that specific movement and the overall focus level and intent to move may be linked automatically to the level (proportionately as previously mentioned) of the specific stimulation for that joint movement. This will further enhance neuroplasticity, and permit the system to help with not only gross movement recovery but fine movements as well. Fine motor movements are those of smaller movements that occur in the wrists, hands, fingers, and the feet and toes. These include actions such as picking up objects between the thumb and finger, grasping objects like cups, or cutting with scissors. In contrast, gross movements come from large muscle groups and whole body movement. Gross movements include actions such as head control, trunk stability, and walking.

By monitoring the brain signals through electrodes, a patient's "engagement metric" may be determined while the patient is thinking about movement. The engagement metric is related to both the focus level and the intent to move, and can be calculated in a number of different ways. In one example, the engagement metric is calculated based on a classification test. In this example, a decoder is trained to identify two motions that a user may perform, motion A and motion B. Then, the user is asked to perform a random series of the motions A and B, and the accuracy of the decoder is determined. The accuracy of the decoder can be used as the engagement metric, as this type of two-class test is difficult to perform if the user is not engaged. As another example, the engagement metric can simply be function of the focus level and the intent to move, weighted as desired.

Based on the engagement metric, the system may then use feedback (e.g. visual/aural) through the game/task that will let the user know they are not engaged in the session. As another alternative, in the games/tasks previously mentioned, the level of difficulty may increase if the user is not engaged, as measured by the engagement metric. For example, the game speed may be increased, or the allotted time for completing a given task may be decreased. Encouraging engagement and use of motor areas of the brain (by using movement related games/tasks) will promote neuroplasticity. Again, use of targeted neuromuscular electrical stimulation (guided by, for example, EEG based metrics) will help patients regain control of desired movements.

It is also advantageous to track patient progress over time. Patient progress may be reported on a PC, laptop, tablet, iPhone, iPad or so forth.

It is contemplated that in particular embodiments, the systems of the present disclosure use a headset that is worn by the patient. The headset includes both EEG electrodes and tDCS electrodes. In tDCS, a constant, low level direct current flows between two or more electrodes. This will further promote neuroplasticity during the rehabilitation.

In some embodiments, an eye tracking device may also be used to determine or aid in determining the patient's focus level or intent to move. The eye tracking device may include a small camera or multiple cameras attached to the headset positioned on the patient. The camera(s) may also be positioned in any other manner so as to as to be able to view the patients eyes (e.g. the camera(s) may be positioned on a desk, positioned on top of a laptop screen, incorporated into a pair of glasses or so forth). In one example, an eye tracking device may determine if the patient's eyes are focused on large virtual body part **320** or small virtual body part **330,** and may use this information as a factor in determining the patient's focus level. In another example, the eye tracking device may be used to determine if the patient's eyes are focused on an actual body part of the patient, either alone or in conjunction with the neural signals, and may use this information to help determine the patient's focus level. For example, when the eye focuses on one thing, the amplitude of the beta frequency band will decrease.

In addition to camera(s) viewing a patient's eyes, camera(s) may also be positioned so that they are pointing outwardly on a pair of glasses that a patient is wearing. This allows a determination to be made of what the patient is looking at, and this determination can be used in conjunction with the observations of the patient's eye movements to calculate a focus level.

A neural sleeve is also used to provide electrical stimulation to a desired body part. The neural sleeve contains a set of electrodes that are used to provide stimulation to the body part when the patient's focus level and intent to move are sufficient. The term "sleeve" is used to refer to a structure that surrounds a body part, for example, an arm, leg, or torso. The neural sleeve can take the form of a shirt, or pants, if desired. The neural sleeve also contains sensors for monitoring movement of the patient (position, orientation, acceleration, etc.), which can be used to track the patient's progress (e.g. their increase in strength or range of motion).

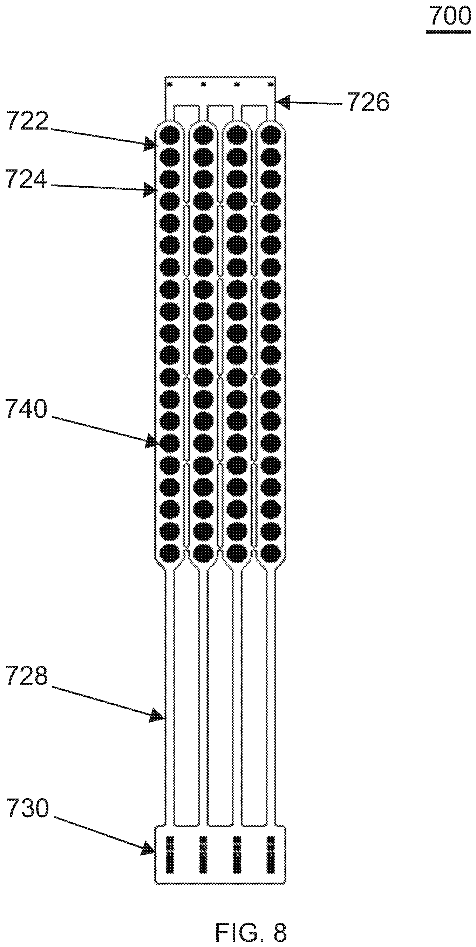

**FIG. 8** is an illustration of one potential neural sleeve that can be used in the methods of the present disclosure. The sleeve **700** as illustrated has an insulating substrate **722** that is shaped into four flexible conductive pathways **710,** each pathway being formed from a finger **724** and a header **728.** The flexible conductive pathways **710** extend in the same direction from the connector **730,** which acts as a connector for one end of the pathways. In other words, the ends of the pathways distal from the connector are all located in the same direction relative to the connector, or put another way the connector **730** is at one end of the device. It is noted that the pathways **710** are shown here as extending at a 90-degree angle relative to the connector **730.** The pathways can be attached to each other, for example by five webbings **725** which run between adjacent fingers **728.**

The electrodes **740** are located or housed on the fingers **724,** and are formed as a layer upon the substrate **722.** The electrodes **740** run along the four fingers **724** and are electrically connected to the connector **730.** The electrodes **740** are approximately 12 mm in diameter and spaced 15 mm apart. A conductive medium, e.g. hydrogel discs, can be laid upon the electrodes to facilitate contact with the user's skin.

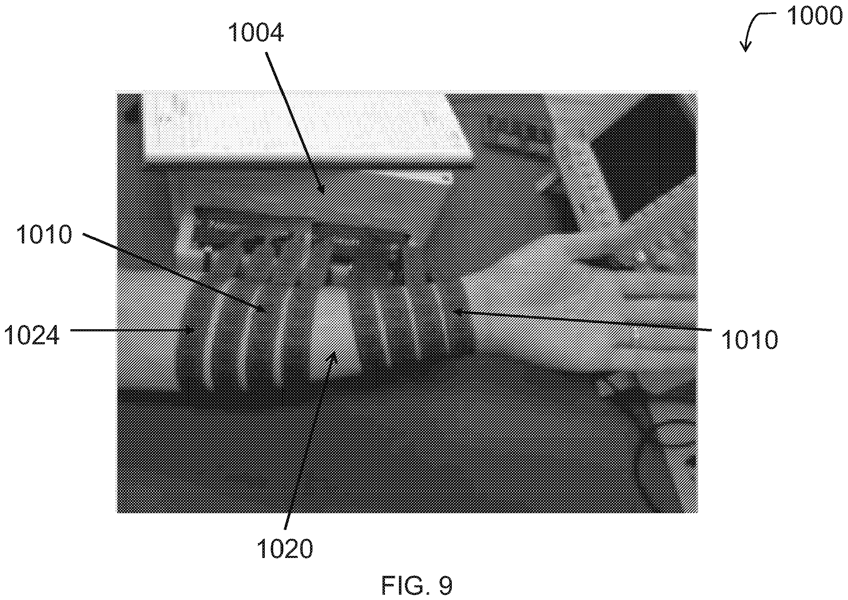

The connector **730** is used for interfacing with the neural signal processor / computer **154 of** **FIG. 1****.** If desired, an optional fork **726** can be located at the end of the pathways opposite the connector **730.** The fork connects all of the fingers, and can be provided for structural support for design and mounting. Headers **728** extend between the connector **730** and the fingers **724.** These headers are thinner than the fingers, and connect the fingers **724** to the connector **730.** The headers are also part of the overall flexible conductive pathway, though they are not always required. Though not illustrated, webbings can also be provided between adjacent headers as well if desired. Again, the fork **726** is optional, though the connector **730** is required. **FIG. 9** shows two neuromuscular cuff devices **1010** of **FIG. 8** being wrapped circumferentially around a patient's arm region **1020** in preparation for neuromuscular stimulation. The two cuff devices **1010** together provide 160 separate electrodes for stimulating finger or wrist movements. The fingers **1024** permit the neuromuscular cuff to fit around the arm region **1020** at points of varying circumference. Hydrogel discs **1016** (not shown) keep both cuffs **1010** adhered to the arm.

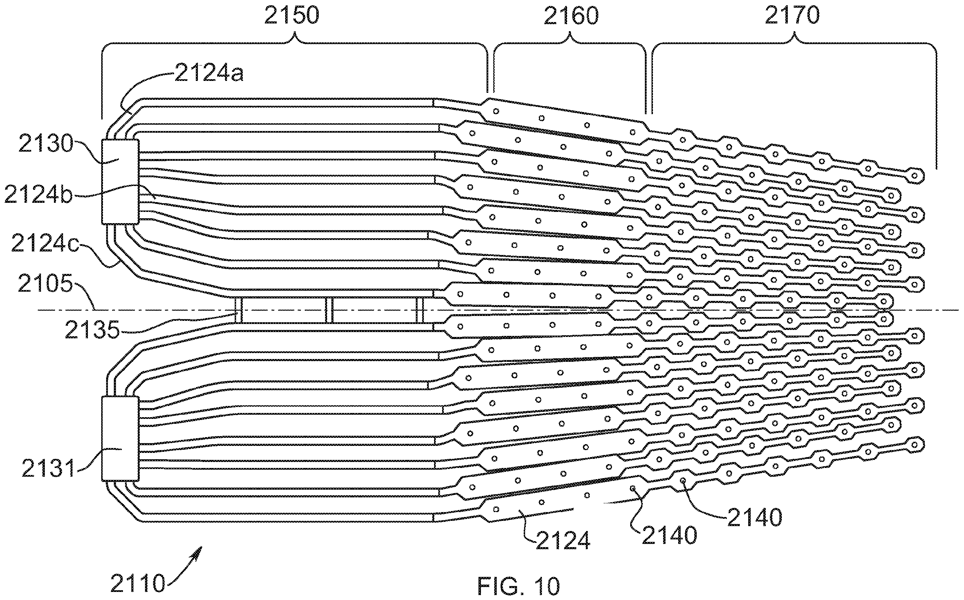

In another exemplary embodiment, the flexible conductive pathways on a neural sleeve **2110** do not need to be straight for their entire length. Referring now to **FIG. 10****,** flexible conductive pathways **2124** extend from first connector **2130,** which has a rectangular shape in this illustration. The flexible conductive pathways **2124** in this embodiment "change" directions as they extend from connector **2130.** For example, an upper flexible conductive pathway **2124a** first extends upwards from the connector **2130,** then changes direction so that its electrodes **2140** are to the right of the connector **2130.** A center flexible conductive pathway **2124b** extends from the right-hand side of the connector **2130** off to the right of the connector. A lower flexible conductive pathway **2124c** first extends downwards from the connector **2130,** then changes direction so that its electrodes **2140** are also to the right of the connector **2130.** Notably, none of the electrodes **2140** are present to the left of the connector **2130.**

This embodiment of a neural sleeve **2110** also contains more than one connector. As illustrated here, the neural sleeve **2110** has a first connector **2130** and a second connector **2131.** Flexible conductive pathways extend in the same direction (here, to the right) of both connectors. Webbings **2135** connect flexible conductive pathways extending from each connector **2130, 2131.** There may be any number of webbings **2135,** and the webbings **2135** may connect the flexible conductive pathways at any portion of their length. Here, the webbings **2135** are present along a a non-electrode-containing portion **2150** of the flexible conductive pathways (i.e. the header portion). Though not depicted, it is specifically contemplated that the flexible conductive pathways of one connector **2130** may be of a different length from the flexible conductive pathways of the other connector **2131.**

The electrodes **2140** may be evenly spaced apart along the length of the flexible conductive pathways **2124,** or their spacing may vary, for example becoming shorter or longer, as the distance from the connector **2130** increases. For example, muscle segments get smaller closer to the wrist, so the electrodes need to be closer together as well. However, the electrodes do not need to be present along the entire length of the flexible conductive pathways. As seen here, the flexible conductive pathways **2124** may include a non-electrode-containing portion **2150** extending from the connector, which is similar to the header **528** of the embodiment of **FIG. 5****.** The flexible conductive pathway may also include a non-scalloped electrode-containing portion **2160,** and a scalloped electrode-containing portion **2170** at the distal end of the flexible conductive pathway (i.e. distal from the connector). It should be noted that none of the flexible conductive pathways overlap with each other.

The electrode-containing portions **2160, 2170** of the flexible conductive pathways have a different shape from each other. One reason for this difference in shape is because, as seen here, the distal ends of the flexible conductive pathways **2124** extend inwardly towards a center axis **2105** of the neural sleeve **2110.** Put another way, the flexible conductive pathways **2124** taper inwards towards the center axis **2105.** The scalloped portions **2170** of adjacent flexible conductive pathways permit them to fit into a smaller area while still providing a suitable number of electrodes (note the electrodes do not change in size). However, the flexible conductive pathways **2124** all still extend in the same direction away from the connector **2130,** i.e. to the right in this figure. Put another way, the flexible conductive pathways comprise a first portion which is transverse to the center axis **2105,** and a second portion which is parallel to the center axis. These portions are particularly seen in the flexible conductive pathway **2124a,** which first extends upwards (i.e. transversely to the center axis), then extends parallel to the center axis.

This particular embodiment is intended to be used on a patient's arm with the two connectors **2130, 2131** located near the shoulder, and the scalloped portions **2170** near the wrist and hand.

In some embodiments as described above, during a rehabilitation session, an EEG headset is worn and used in conjunction with the neural sleeve. In other embodiments, it is contemplated that the neural sleeve can used without an EEG headset (referred to as ambulatory mode). In these embodiments, EMG and/or other sensing can be used by the neural sleeve to amplify weak muscle signals or to provide strength or assistance in desired movements. The neural sleeve may have built-in voice recognition as well. In such embodiments, the neural sleeve is used by itself to provide rehabilitative sensing and stimulation. A patient whose nerves in the affected body part are merely weak could use the neural sleeve to strengthen the body part and accelerate recovery.

It is noted that the same electrodes used for stimulation in the neural sleeve can also be used for recording (i.e. EMG). As the muscles get stronger, this can be detected by the strength of the EMG signal. When the electrodes are not stimulating, the neural sleeve can be used to measure a baseline EMG signal or a baseline range of motion. This recording period can last from about 5 minutes to about 10 minutes. In particular embodiments, the electrical stimulation delivered to the muscles is a function of the patient's measured strength. As another example, when a patient's measured strength exceeds a predetermined threshold, this can be another trigger to decrease the electrical stimulation delivered to the neural sleeve and the muscles, i.e. the rehabilitation is tapering off. The predetermined threshold can change over time, and a new baseline can be periodically measured to make such determinations. A stimulation factor can be used to determine the strength of the electrical stimulation based on any of several variables, such as time of rehabilitation, measured muscle strength, etc.

In yet another aspect, referred to as mirror mode, a movement may be sensed on one side of a body and stimulated on another side of the body. For example, when a patient moves a right hand, the system may stimulate a movement in the patient's left hand. This may be advantageous when, for example, a stroke has affected one side of the body.

It is also contemplated that the neural sleeve may be wireless (e.g. communicate with computer **154** wirelessly). The neural sleeve may also be battery-based. This could be accomplished, for example, by strapping a battery or control pack in an iPhone-like carrier to a patient arm, patient leg, belt, or so forth.

It will further be appreciated that the disclosed techniques may be embodied as a non-transitory storage medium storing instructions readable and executable by a computer, (microprocessor or microcontroller of an) embedded system, or various combinations thereof. The non-transitory storage medium may, for example, comprise a hard disk drive, RAID or the like of a computer; an electronic, magnetic, optical, or other memory of an embedded system, or so forth.

The preferred embodiments have been illustrated and described. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A system for rehabilitating a patient with motor impairment, comprising:
electrodes positioned on or in the patient's head;
a processing unit connected to receive neural signals from the electrodes; and
sleeve electrodes of a sleeve positioned on a specific body part of the patient; wherein
said electrodes are capable of sensing neural signals from the brain of the patient;
said processing unit is capable of receiving electrical signals from the electrodes positioned on or in the patient's head; capable of determining an intent to move a specific body part of the patient from the electrical signals; and capable of determining a focus level of the patient from the electrical signals; and
based on the intent to move and focus level, said processing unit is capable of delivering electrical stimulation through said sleeve electrodes to cause movement of the specific body part,
wherein the intent to move is calculated based on a change in amplitude of the beta frequency band in the received electrical signals.

2. The system of claim 1, wherein the amplitude of the beta frequency band decreases by at least 5% for a time of at least one-tenth second.

3. The system of claim 1, wherein the intent to move is calculated after the patient goes through a calibration period of about five seconds to about 20 seconds to determine a baseline by which changes are measured.

4. The system of claim 1, wherein the focus level is determined by a change in amplitude of the gamma frequency band in the received electrical signals compared to a calibration period.

5. The system of claim 4, wherein the amplitude of the gamma frequency band increases by at least 5% for a time of at least one-tenth second.

6. The system of claim 1, wherein said processing unit is further capable of determining that the patient is focusing on a desired movement of the specific body part, or
further capable of engaging the patient with a rehabilitative videogame that instructs the patient to move an impaired body part of the patient, or
further capable of using transcranial direct current stimulation (tDCS) with the patient.

7. The system of claim 1, wherein the amplitude of the delivered electrical stimulation to the specific body part is a function of at least one feature of the received electrical signals.

8. The system of claim 7, wherein the at least one feature is selected from the group consisting of the signal amplitude, the amplitude of the signal in a given frequency range, the amplitude of the signal in a wavelet scale, and the firing rate, or
wherein the delivered electrical stimulation is decreased based on a change in strength of the patient.

9. The system of claim 1, further comprising:
tracking an eye movement of the patient with an eye tracking device; and
determining the focus level based on the electrical signals from the electrodes and the tracked eye movement.

10. The system of claim 9, wherein the eye tracking device comprises a camera connected to the headset.

11. The system of claim 9, wherein the determined focus level of the patient is modified based on whether an eye of the patient is focused on the specific body part of the patient.

12. The system of claim 10, wherein said processing unit is further capable of increasing a difficulty level of the videogame if the focus level is below a predetermined threshold, or
said processing unit is further capable of:
calculating a stimulation factor based on the electrical signals during a first rehabilitative session;
reducing the calculated stimulation factor prior to a second rehabilitative session; and
delivering the electrical stimulation based on the calculated stimulation factor.

13. The system of claim 10, wherein if the focus level is below a predetermined threshold, providing a visual or aural indication that the patient is not focused.

14. The system of claim 1, wherein the received electrical signals are received from implanted electrodes implanted within the patient.

## Patentansprüche

1. System zum Rehabilitieren eines Patienten mit motorischer Beeinträchtigung, umfassend:
Elektroden, die auf oder in dem Kopf des Patienten positioniert sind;
eine Verarbeitungseinheit, die verbunden ist, um neuronale Signale von den Elektroden zu empfangen; und
Manschettenelektroden [Englisch: sleeve electrodes) einer Manschette, die auf einem spezifischen Körperteil des Patienten positioniert ist; wobei die Elektroden in der Lage sind, neuronale Signale vom Gehirn des Patienten zu erfassen;
die Verarbeitungseinheit in der Lage ist, elektrische Signale von den Elektroden zu empfangen, die auf oder in dem Kopf des Patienten positioniert sind; in der Lage ist, aus den elektrischen Signalen eine Absicht zu bestimmen, einen spezifischen Körperteil des Patienten zu bewegen; und in der Lage ist, aus den elektrischen Signalen ein Fokusniveau des Patienten zu bestimmen; und
basierend auf der Absicht, sich zu bewegen, und dem Fokusniveau, die Verarbeitungseinheit in der Lage ist, eine elektrische Stimulation durch die Manschettenelektroden zu liefern, um eine Bewegung des spezifischen Körperteils zu bewirken,
wobei die Absicht, sich zu bewegen, basierend auf einer Änderung in der Amplitude des Beta-Frequenzbandes in den empfangenen elektrischen Signalen berechnet wird.

2. System nach Anspruch 1, wobei die Amplitude des Beta-Frequenzbandes für eine Zeit von mindestens einer Zehntelsekunde um mindestens 5 % abnimmt.

3. System nach Anspruch 1, wobei Absicht, sich zu bewegen, berechnet wird, nachdem der Patient einen Kalibrierungszeitraum von etwa fünf Sekunden bis etwa 20 Sekunden durchlaufen hat, um eine Basislinie zu bestimmen, an der Änderungen gemessen werden.

4. System nach Anspruch 1, wobei das Fokusniveau durch eine Änderung in der Amplitude des Gamma-Frequenzbandes in den empfangenen elektrischen Signalen im Vergleich zu einem Kalibrierungszeitraum bestimmt wird.

5. System nach Anspruch 4, wobei die Amplitude des Gamma-Frequenzbandes für eine Zeit von mindestens einer Zehntelsekunde um mindestens 5 % zunimmt.

6. System nach Anspruch 1, wobei die Verarbeitungseinheit ferner in der Lage ist, zu bestimmen, dass sich der Patient auf eine gewünschte Bewegung des spezifischen Körperteils fokussiert, oder
ferner in der Lage ist, den Patienten mit einem rehabilitativen Videospiel zu beschäftigen, das den Patienten anweist, einen beeinträchtigten Körperteil des Patienten zu bewegen, oder
ferner in der Lage ist, die transkraniale Gleichstromstimulation (tDCS, für Englisch "transcranial direct current stimulation") mit dem Patienten zu verwenden.

7. System nach Anspruch 1, wobei die Amplitude der gelieferten elektrischen Stimulation an den spezifischen Körperteil eine Funktion von mindestens einem Merkmal der empfangenen elektrischen Signale ist.

8. System nach Anspruch 7, wobei das mindestens eine Merkmal aus der Gruppe gewählt ist, bestehend aus der Signalamplitude, der Amplitude des Signals in einem gegebenen Frequenzbereich, der Amplitude des Signals in einer Wavelet-Skala und der Feuerungsrate, oder
wobei die gelieferte elektrische Stimulation basierend auf einer Änderung in der Stärke des Patienten verringert wird.

9. System nach Anspruch 1, ferner umfassend:
Verfolgen einer Augenbewegung des Patienten mit einer Augenverfolgungsvorrichtung; und
Bestimmen des Fokusniveaus basierend auf den elektrischen Signalen von den Elektroden und der verfolgten Augenbewegung.

10. System nach Anspruch 9, wobei die Augenverfolgungsvorrichtung eine Kamera umfasst, die mit dem Headset verbunden ist.

11. System nach Anspruch 9, wobei das bestimmte Fokusniveau des Patienten modifiziert wird, basierend darauf, ob ein Auge des Patienten auf den spezifischen Körperteil des Patienten fokussiert ist.

12. System nach Anspruch 10, wobei die Verarbeitungseinheit ferner in der Lage ist, einen Schwierigkeitsgrad des Videospiels zu erhöhen, wenn das Fokusniveau unter einem vorbestimmten Schwellenwert liegt, oder
die Verarbeitungseinheit ferner zu dem Folgenden in der Lage ist:
Berechnen eines Stimulationsfaktors basierend auf den elektrischen Signalen während einer ersten rehabilitativen Sitzung;
Reduzieren des berechneten Stimulationsfaktors vor einer zweiten rehabilitativen Sitzung; und
Bereitstellen der elektrischen Stimulation basierend auf dem berechneten Stimulationsfaktor.

13. System nach Anspruch 10, wobei, wenn das Fokusniveau unter einem vorbestimmten Schwellenwert liegt, eine visuelle oder akustische Anzeige bereitgestellt wird, dass der Patient nicht fokussiert ist.

14. System nach Anspruch 1, wobei die empfangenen elektrischen Signale von implantierten Elektroden empfangen werden, die in den Patienten implantiert sind.

## Revendications

1. Système de rééducation d'une déficience motrice d'un patient, comprenant :
des électrodes positionnées sur ou dans la tête du patient ;
une unité de traitement connectée de manière à recevoir des signaux neuronaux en provenance des électrodes ; et
des électrodes à manchon d'un manchon positionné sur une partie corporelle spécifique du patient ; dans lequel
lesdites électrodes sont en mesure de détecter des signaux neuronaux provenant du cerveau du patient ;
ladite unité de traitement est en mesure de recevoir des signaux électriques provenant des électrodes positionnées sur ou dans la tête du patient ; elle est en mesure de déterminer une intention de déplacer une partie corporelle spécifique du patient, à partir des signaux électriques ; et elle est en mesure de déterminer un niveau de concentration du patient à partir des signaux électriques ; et
sur la base de l'intention de déplacement et du niveau de concentration, ladite unité de traitement est en mesure de fournir une stimulation électrique à travers lesdites électrodes à manchon en vue de provoquer un mouvement de la partie corporelle spécifique ;
l'intention de déplacement étant calculée sur la base d'un changement d'amplitude de la bande de fréquence bêta dans les signaux électriques reçus.

2. Système selon la revendication 1, dans lequel l'amplitude de la bande de fréquence bêta diminue d'au moins 5 % pendant une durée d'au moins un dixième de seconde.

3. Système selon la revendication 1, dans lequel l'intention de déplacement est calculée suite à une période d'étalonnage du patient, d'environ cinq secondes à environ 20 secondes, pour déterminer une référence par laquelle des changements sont mesurés.

4. Système selon la revendication 1, dans lequel le niveau de concentration est déterminé par un changement d'amplitude de la bande de fréquence gamma dans les signaux électriques reçus, par rapport à une période d'étalonnage.

5. Système selon la revendication 4, dans lequel l'amplitude de la bande de fréquence gamma augmente d'au moins 5 % pendant une durée d'au moins un dixième de seconde.

6. Système selon la revendication 1, dans lequel ladite unité de traitement est en outre en mesure de déterminer que le patient se concentre sur un mouvement souhaité de la partie corporelle spécifique ; ou
elle est en outre en mesure d'impliquer le patient dans un jeu vidéo de rééducation qui donne instruction au patient de déplacer une partie corporelle altérée du patient ; ou
elle est en outre en mesure d'utiliser une stimulation transcrânienne en courant continu (tDCS) pour le patient.

7. Système selon la revendication 1, dans lequel l'amplitude de la stimulation électrique fournie à la partie corporelle spécifique est en fonction d'au moins une caractéristique des signaux électriques reçus.

8. Système selon la revendication 7, dans lequel ladite au moins une caractéristique est sélectionnée à partir du groupe constitué par l'amplitude du signal, l'amplitude du signal dans une plage de fréquences donnée, l'amplitude du signal dans une échelle d'ondelettes, et le taux de décharge ; ou
dans lequel la stimulation électrique fournie est diminuée sur la base d'une variation de l'effort du patient.

9. Système selon la revendication 1, comprenant en outre :
le suivi d'un mouvement oculaire du patient à l'aide d'un dispositif de suivi oculaire ; et
la détermination du niveau de concentration sur la base des signaux électriques provenant des électrodes et du mouvement oculaire suivi.

10. Système selon la revendication 9, dans lequel le dispositif de suivi oculaire comprend une caméra connectée au casque d'écoute.

11. Système selon la revendication 9, dans lequel le niveau de concentration déterminé du patient est modifié selon qu'un oeil du patient est concentré sur la partie corporelle spécifique du patient.

12. Système selon la revendication 10, dans lequel ladite unité de traitement est en outre en mesure d'augmenter un niveau de difficulté du jeu vidéo si le niveau de concentration est inférieur à un seuil prédéterminé ; ou
ladite unité de traitement est en outre en mesure de :
calculer un facteur de stimulation sur la base des signaux électriques lors d'une première séance de rééducation ;
réduire le facteur de stimulation calculé avant une seconde séance de rééducation ; et
fournir la stimulation électrique sur la base du facteur de stimulation calculé.

13. Système selon la revendication 10, dans lequel, si le niveau de concentration est inférieur à un seuil prédéterminé, une étape consistant à fournir une indication visuelle ou sonore indiquant que le patient n'est pas concentré est mise en oeuvre.

14. Système selon la revendication 1, dans lequel les signaux électriques reçus sont reçus en provenance d'électrodes implantées lesquelles sont implantées à l'intérieur du patient.
